# EUROPEAN PATENT SPECIFICATION

(11) **EP 0 301 897 B1**
(45) Date of publication and mention of the grant of the patent: **26.10.1994**
(21) Application number: 88307035.1
(22) Date of filing: 29.07.1988
(51) Int. Cl.: G01N 27/28

(54) **Oxygen analyzer**
Sauerstoffanalysengerät
Analyseur d'oxygène

(30) Priority: 31.07.1987 JP 192415/87; 31.07.1987 JP 192416/87
(43) Date of publication of application: 01.02.1989
(60) Divisional application: 92117486.8
(73) Proprietor: Osaka Sanso Kogyo Limited, Yodogawa-ku Osaka (JP)
(72) Inventor: Makihara, Akira, Funabashi-shi Chiba-ken (JP); Matsumoto, Yoshiro, Funabashi-shi Chiba-ken (JP); Kikuchi, Shigeru, Kamagaya-shi Chiba-ken (JP)
(74) Representative: Cropp, John Anthony David

(56) References cited:
- EP-A- 0 218 469
- FR-A- 1 397 609
- FR-A- 2 111 421
- FR-A- 2 415 298
- GB-A- 896 290
- GB-A- 1 272 619
- PATENT ABSTRACTS OF JAPAN, vol. 10, no. 206 (P-478)(2262), 18 July 1986;& JP-A-61047548

## Description

The present invention relates to a galvanic cell type oxygen analyzer. More particularly, the present invention pertains to a galvanic cell type oxygen analyzer wherein a specific noble metal is employed as a material for an anode protecting electrode in order to reduce noise and obtain a stable output.

The present invention is also concerned with an oxygen analyzer wherein a specific metal is employed as a material for a water electrolyzing electrode of a standard gas generator for water electrolysis type calibration.

Generally, hydrogen is obtained by electrolysis of water or in the form of a gas produced as a by-product by iron works. When such a hydrogen gas is employed in the manufacture of semiconductor devices, a trace amount of oxygen contained in the hydrogen gas may impair the production of semiconductor devices. It is, therefore, necessary to measure the oxygen content in the hydrogen gas. It has heretofore been general practice to employ a galvanic cell type oxygen analyzer to measure the O₂ content in the hydrogen gas.

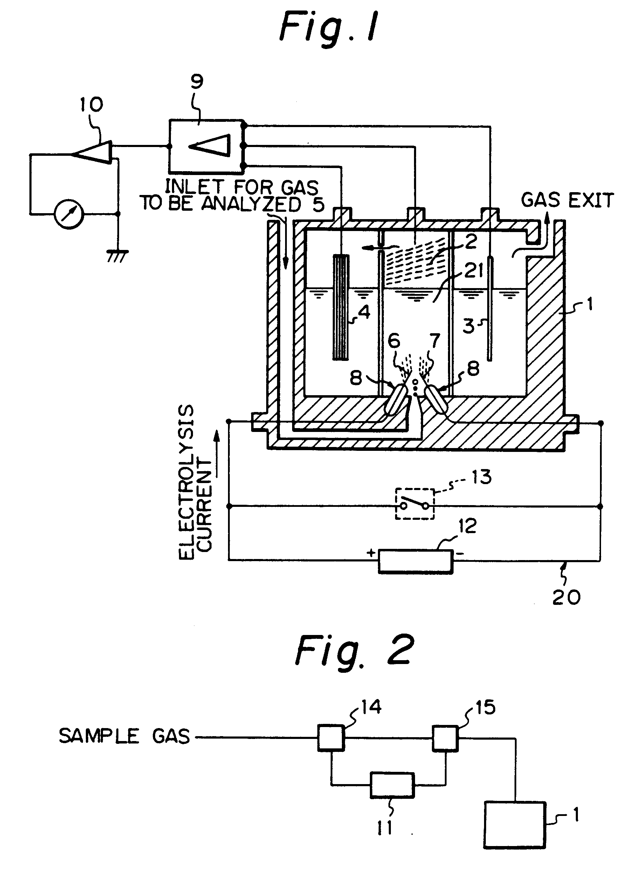

Fig. 1 schematically shows a known galvanic cell type oxygen analyzer. The following is a description of the principle of this galvanic cell type oxygen analyzer.

An analyzer cell 1 is a kind of galvanic cell which consists of a potassium hydroxide (20% KOH) electrolyte, and an inert silver (Ag) cathode 2, a protecting electrode 3 and an active cadmium (Cd) anode 4, all of which are immersed in the electrolyte. The silver electrode 2 is composed of about ten odd spirally arranged silver disks which are covered with a cylindrical tube. The cadmium electrode 4 has a relatively long and narrow cylindrical configuration. The protecting electrode 3 has a relatively long and narrow ribbon-shaped configuration. When a sample gas is supplied to this cell from a gas inlet 5, the gas passes through a wet tube in the center of the cell 1 to reach the surface of the silver electrode 2, and an amount of oxygen which is proportional to the oxygen partial pressure in the gas is adsorbed on the surface of the silver electrode 2. The adsorbed oxygen molecules dissolve in the electrolyte in the form of hydroxyl ions.

### [Silver cathode]

O₂ + 2H₂O + 4e → 4OH⁻ (1)

The hydroxyl ions move toward the cadmium anode 4 and oxidize cadmium to form cadmium hydroxide.

### [Cadmium anode]

2Cd + 4OH⁻ → 2Cd(OH)₂ + 4e (2)

When the hydroxyl ions lose their negative charges in this way, a current flows from the silver electrode 2 to an external circuit connected to the cadmium electrode 4. Accordingly, by measuring this current, it is possible to measure the oxygen concentration in the gas.

When the oxygen concentration is particularly high, the reactions of the formulae (1) and (2) proceed rapidly, so that the surface of the cadmium electrode 4 is oxidized quickly, resulting in lowering of the anode capacity. The protecting electrode 3 is provided in order to protect the cadmium electrode 4 from oxidation.

The protecting electrode 3 is supplied with the current flowing between the silver electrode 2 and the cadmium electrode 4 after it has been amplified.

Accordingly, most of the hydroxyl ions generated from the reaction of the formula (1) are attracted by the protecting electrode 3. Since the relatively small current flowing between the silver electrode 2 and the cadmium electrode 4 and the relatively large current flowing to the protecting electrode 3 are in proportional relation to each other, the current flowing to the protecting electrode 3 is utilized as an electric output from the cell 1.

In conventional galvanic cell type oxygen analyzers which have an anode protecting electrode, platinum, which is resistant to acids and alkalis, is employed as a material for the protecting electrode. However, it has been confirmed that the level of output noise increases somewhat when a trace amount of oxygen contained in a gas rich in hydrogen is measured with an oxygen analyzer employing platinum as a material for the anode protecting electrode. The smaller the oxygen content, the higher the noise level. Accordingly, the detectable minimum limit for the oxygen concentration in gases which are rich in hydrogen has heretofore been somewhat lower than that in the case of other inert gases (e.g., helium, argon, nitrogen, etc.) or combustible gases (methane, ethane, ethylene, etc.).

In general, calibration of a gas analyzer is effected using a zero gas filled in a vessel and a standard gas. In the case of oxygen standard gases, particularly those which contain a trace amount of oxygen, i.e., 1 ppm or less, the degree of precision lowers with the passage of time due to reaction with the surface of the vessel, degassing, etc. There is one type of trace oxygen analyzer generally known which has a purifier for removing the oxygen content from a gas which is to be measured to thereby obtain a zero gas and a standard gas generator for generating a standard oxygen gas by electrolysis of water. This type of oxygen analyzer suffers, however, from the disadvantage that it may be difficult or impossible to generate pure oxygen stably in a trace oxygen region depending upon the kind of sample gas employed.

When H₂, N₂ or Ar is employed as a zero gas in a calibration oxygen generator, there has heretofore been a risk that O₂ may not be normally generated from the anode of the oxygen analyzer. If platinum or the like is, by way of example, employed as a material for the anode or cathode of the oxygen generator, the amount of O₂ generated is reduced, although the mechanism responsible for this is not clear, so that it is impossible to obtain an O₂ gas for calibration having a desired concentration.

It is conventional practice from the viewpoint of thermal expansion to secure a platinum wire which serves as the anode or cathode of the oxygen analyzer through a soft glass. However, where the platinum wire is secured to the bottom of a detector cell, since an alkaline solution such as a potassium hydroxide, sodium hydroxide or potassium hydrogencarbonate solution is used as an electrolyte, the soft glass elutes with the passage of time, resulting in a gap at the interface between the glass and the platinum wire. The pure oxygen that is generated at this gap may form bubbles; in such a case, every time bubbles are liberated, the predetermined dilution ratio is destroyed, which makes it impossible to obtain a stable-state standard gas. There are cases where a hard glass which is considered to be only slightly soluble in an alkali is employed. However, in many cases, a crack develops at the interface between the glass and the platinum wire at the time of fusion welding due to the difference in the thermal expansion coefficients. Therefore, the production yield is low, and there is a fear of bubbles being generated, as described above.

On the other hand, there is a residual voltage between the anode and the cathode when no electrolysis is conducted, which promotes adhesion to the electrode surface of impurity metal ions in the water or the diluent gas, resulting in a film of impurities forming on the electrode surface. The resulting film of impurities obstructs normal electrolysis in a low oxygen concentration region.

The present inventors have found that the conventional anode protecting electrode formed by using platinum or the like readily causes dissociation and adsorption of hydrogen during analysis of a gas which is rich in hydrogen, resulting in an increase in the level of noise.

The present inventors conducted extensive studies and have found that employment of gold, silver or rhenium as one of the constituent materials of the anode protecting electrode of an oxygen analyzer enables a reduction in the noise that results from the dissociation and adsorption of hydrogen. The present invention has been accomplished on the basis of this finding.

GB-A-2013895 describes an electroanalytical cell for amperometric measurement of an electroactive component, such as oxygen, in a gas or liquid. The cell has three electrodes, one of which is present to trap impurities in the electrolyte solution to prevent the impurities contributing to the amperometric measurement of the cell.

FR-A-1397609 also describes an amperometric cell and containing three anodes, one of the anodes functioning to collect impurities and prevent build up of deposits on the other anodes.

JP-A-61047548 describes a gas detection element comprising a neutral third electrode between the detector electrodes.

The present invention provides an oxygen analyzer comprising a galvanic cell for use together with associated circuitry and adapted to analyze oxygen in a hydrogen rich gas, said cell comprising first and second measuring electrodes acting respectively as an anode and a cathode, and at least one supplementary electrode arranged to intercept hydroxyl ions generated at the cathode so as to protect the anode from oxidation, characterised in that said supplementary electrode comprises an anode protecting electrode at least a surface portion of which comprises gold, silver or rhenium.

This invention also provides, in an embodiment, an oxygen analyzer wherein the supplmentary electrode comprises a water electrolysing electrode of a standard gas generator of the water electrolysis type.

This invention further provides a method of analyzing oxygen in a hydrogen-rich gas which comprises detecting the potential difference between an anode and a cathode in contact with an electrolyte exposed to said gas, characterized in that at least one supplementary electrode is provided in contact with the electrolyte and arranged to intercept hydroxyl ions generated at the cathode so as to protect the anode from oxidation wherein at least a surface portion of said supplementary electrode comprises gold, silver or rhenium.
Fig. 1 schematically shows a galvanic cell type oxygen analyzer;
Fig. 2 schematically shows a system for feeding a sample gas;
Fig. 3 is a graph, showing the noise level in the case of an anode protecting electrode formed using platinum; and
Fig. 4 is a graph showing the noise level in the case of an anode protecting electrode formed using gold.

Preferable examples of the material for the anode protecting electrode of an oxygen analyzer or a water electrolyzing electrode of a standard gas generator for water electrolysis type calibration are gold, silver and rhenium. Among these, gold is the most preferable. Accordingly, it is preferable to make such electrodes of gold, silver or rhenium, or to use electrode materials which are formed by coating the reaction surface or the whole surface of a metal (e.g., copper or nickel), ceramic or resin material with gold, silver or rhenium by means, for example, of sputtering. These metals may, of course, be coated by other methods.

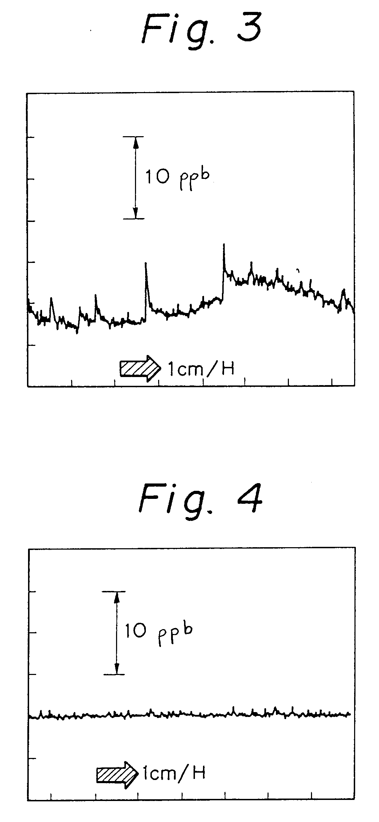

An anode protecting electrode was experimentally formed using gold, and the noise level was measured in the range of 100 ppb. The noise level in the case of the gold anode-protecting electrode was ±1 ppb or less, whereas the noise level in the case of a platinum anode-protecting electrode was about ±5 ppb. The results of the measurement are shown in Figs. 3 and 4. Fig. 3 is a graph showing the noise level in the case of the platinum anode-protecting electrode, while Fig. 4 is a graph showing the noise level in the case of the gold anode-protecting electrode. As will be understood from these graphs, employment of gold as a material for the anode protecting electrode enables the noise level to be lowered to about one fifth of that in the case of a platinum anode-protecting electrode.

As described above, the present invention enables the noise generated in the measurement of oxygen contained in a trace amount in a gas rich in hydrogen to be lowered to the same level as in the case of other inert gases or combustible gases. Thus, it is possible to measure precisely the concentration of oxygen contained in a trace amount in a gas which is rich in hydrogen.

It should be noted that the arrangement shown in Fig. 1 is also provided with a calibration circuit 20 for generating an oxygen standard gas by electrolysis of water. The reference numeral 6 denotes an anode, 7 a cathode, 8 epoxy resin sleeves, and 9, 10 amplifiers.

Fig. 2 shows a system for feeding a sample gas for measurement. At the time of analysis of O₂, the sample gas is not passed through an O₂ purifier 11 but is supplied to the gas inlet 5 of the cell 1 through valves 14 and 15. When calibration of the oxygen analyzer is to be conducted, the sample gas is passed through the O₂ purifier 11 where oxygen is completely removed, and then introduced into the cell 1 from the inlet 5 as an "oxygen zero gas". The reference numeral 12 denotes a power supply, and 13 a switch. When a current is passed through the circuit 20, O₂ and H₂ are generated from the anode 6 and the cathode 7, respectively. The pure O₂ thus generated is diluted with the oxygen zero gas introduced from the gas inlet 5 to thereby generate a standard gas having a predetermined oxygen concentration.

Referring to Fig. 1, the oxygen analyzer according to the present invention comprises a pair of water electrolyzing electrodes, epoxy resin sleeves, and a switch for shorting these two electrodes. The present invention will be described hereinunder in detail with reference to Fig. 1 which shows the structure of the oxygen analyzer. The reference numerals 6 and 7 denote a pair of water electrolyzing electrodes. That portion of each of the electrodes 6 and 7 which is in contact with an electrolyte is formed using gold, silver or rhenium, preferably gold, which is the noblest metal. The electrodes 6 and 7 may have any kind of configuration, e.g., a linear, rod- or band-shaped configuration. The distal end of each electrode may be coiled. It is also possible to form these electrodes 6 and 7 by coating an electrically conductive material, e.g., copper or nickel, with gold, silver or rhenium by means of, for example, sputtering, vacuum evaporation or electroplating. Osmium causes less dissociation and adsorption of hydrogen but has inferior workability and is therefore unsuitable for use as an electrode material. If an electrolytic current is passed in the illustrated direction, pure oxygen and pure hydrogen are generated by the electrodes 6 and 7, respectively. The pure oxygen generated by the electrode 6 is diluted with the oxygen zero gas introduced from the inlet 5 to thereby obtain an oxygen standard gas. When hydrogen is, by way of example, employed as an oxygen zero gas, an electrode formed from gold, silver or rhenium is unlikely to cause dissociation and adsorption of hydrogen at its surface and therefore exhibits excellent water electrolysis characteristics in a trace oxygen region. In addition, the period of time from the instant the application of a constant current is started until the generation of pure oxygen begins is shorter than that in the case of an electrode formed using platinum or other platinum-based noble alloys, and therefore the electrode of gold, silver or rhenium exhibits excellent stability.

The reference numeral 1 denotes a vessel for electrolysis of water which may also serve as a detector cell and/or humidifier. The vessel 1 is formed from a material which is selected from the group consisting of epoxy resins, vinyl chloride and hard glass, preferably an epoxy resin which adheres most strongly to the epoxy resin sleeves 8 from the viewpoint of such aspects as resistance to alkalis, gas barrier properties and electrical insulating properties.

The reference numeral 21 denotes an alkaline solution such as a potassium hydroxide, sodium hydroxide or potassium hydrogencarbonate solution.

The numeral 8 denotes epoxy resin sleeves. An electrode material is received through each cylindrical epoxy resin sleeve 8 and bonded thereto by means of an epoxy resin adhesive. Further, the epoxy resin sleeves 8 are secured to the vessel 1 for electrolysis of water by means of an epoxy resin adhesive. Many epoxy resins have their setting temperatures adjusted to ordinary temperature by being mixed with a hardener. Therefore, employment of an epoxy resin as a material for the vessel 1 for water electrolysis and the sleeves 8 requires no heating or cooling steps in the process for securing the electrodes. Accordingly, no cracks are generated at the interface between the electrode material and the epoxy resin which would otherwise be developed due to the difference in thermal expansion coefficients. In other words, the pure oxygen which is generated by electrolysis of water is effectively diffused into the electrolyte without producing bubbles.

The reference numeral 13 denotes a switch. The switch 13 is provided on the circuit between the anode and the cathode which are defined by the water electrolyzing electrodes. When electrolysis is being conducted, the switch 13 is opened, whereas when no electrolysis is taking place, the switch 13 is closed. Thus, there is no residual voltage between the two electrodes when no electrolysis is being conducted. Accordingly, it is possible to prevent adhesion of impurity metal ions to the electrode surface, and it becomes easy to conduct water electrolysis in a trace oxygen region.

The reference numeral 20 denotes a constant-current circuit.

The present invention enables pure oxygen to be readily and stably generated in a trace oxygen region even in the case of a hydrogen-rich diluent gas. In addition, it is possible to prevent production of bubbles of pure oxygen at the interface between the electrode material and the epoxy resin sleeve, and therefore there is no risk of the predetermined dilution ratio being destroyed. Accordingly, it is possible to obtain a standard gas having a stable oxygen concentration. It is also possible to prevent the formation of a film on the electrode surface by impurity metal ions, and it is therefore possible to conduct stable electrolysis of a hydrogen-rich gas which contains a trace amount of oxygen.

## Claims

1. An oxygen analyzer comprising a galvanic cell with associated circuitry and being adapted to analyze oxygen in a hydrogen rich gas, said cell comprising first and second measuring electrodes acting respectively as an anode and a cathode, and at least one supplementary electrode arranged to intercept hydroxyl ions generated at the cathode so as to protect the anode from oxidation, characterised in that said supplementary electrode comprises an anode protecting electrode at least a surface portion of which comprises gold, silver or rhenium.

2. An oxygen analyzer according to Claim 1, wherein said supplementary electrode comprises a water electrolyzing electrode of a standard gas generator of the water electrolysis type.

3. An oxygen analyzer according to Claim 1 or Claim 2, wherein the surface portion or the whole surface of said anode protecting electrode is coated with gold, silver or rhenium by means of sputtering, vacuum evaporation or plating.

4. A method of analyzing oxygen in a hydrogen-rich gas which comprises detecting the potential difference between an anode and a cathode in contact with an electrolyte exposed to said gas, wherein there is provided at least one supplementary electrode in contact with the electrolyte and arranged to intercept hydroxyl ions generated at the cathode so as to protect the anode from oxidation, characterized in that at least a surface portion of said supplementary electrode comprises gold, silver or rhenium.

## Patentansprüche

1. Sauerstoffanalysator mit einer Galvanikzelle mit einer zugeordneten Schaltung, der zur Analyse von Sauerstoff in einem wasserstoffreichen Gas ausgebildet ist, wobei die Zelle erste und zweite Meßelektroden aufweist, die als Anode bzw. als Kathode wirken, und zumindest eine Hilfselektrode, die so angeordnet ist, daß sie Hydroxylionen abfängt, die an der Kathode erzeugt werden, um so die Anode gegen eine Oxidation zu schützen,
dadurch **gekennzeichnet,** daß die
Hilfselektrode eine Anoden-Schutzelektrode aufweist, bei welcher zumindest ein Oberflächenabschnitt Gold, Silber oder Rhenium aufweist.

2. Sauerstoffanalysator nach Anspruch 1,
bei welchem die Hilfselektrode eine
Wasserelektrolyseelektrode eines Standardgasgenerators des Wasserelektrolysetyps aufweist.

3. Sauerstoffanalysator nach Anspruch 1 oder Anspruch 2, bei welchem der Oberflächenabschnitt oder die gesamte Oberfläche der Anoden-Schutzelektrode mit Gold, Silber oder Rhenium mittels Sputtern, Vakuumverdampfung oder Plattieren beschichtet ist.

4. Verfahren zur Analyse von Sauerstoff in einem wasserstoffreichen Gas, bei welchem die Potentialdifferenz zwischen einer Anode und einer Kathode in Berührung mit einem Elektrolyten, welcher dem Gas ausgesetzt ist, erfaßt wird, wobei zumindest eine Hilfselektrode in Berührung mit dem Elektrolyten vorgesehen ist, die so angeordnet ist, daß sie Hydroxylionen abfängt, die an der Kathode erzeugt werden, um so die Anode gegen Oxidation zu schützen, dadurch **gekennzeichnet,** daß zumindest ein Oberflächenabschnitt der Hilfselektrode Gold, Silber oder Rhenium aufweist.

## Revendications

1. Un analyseur d'oxygène comprenant une cellule galvanique à système de circuit associé et destinée à analyser l'oxygène dans un gaz riche en hydrogène, ladite cellule comprenant des première et seconde électrodes de mesure agissant respectivement comme anode et comme cathode et au moins une électrode supplémentaire agencée pour intercepter des ions hydroxyle engendrés à la cathode, de façon à protéger l'anode contre l'oxydation, caractérisé en ce que ladite électrode supplémentaire comprend une électrode protectrice d'anode dont au moins une portion de surface comprend de l'or, de l'argent ou du rhénium.

2. Un analyseur d'oxygène selon la revendication 1, dans lequel ladite électrode supplémentaire comprend une électrode d'électrolyse d'eau d'un générateur de gaz normalisé du type à électrolyse d'eau.

3. Un analyseur d'oxygène selon la revendication 1 ou la revendication 2, dans lequel la portion de surface ou toute la surface de ladite électrode protectrice d'anode est revêtue d'or, d'argent ou de rhénium au moyen d'une pulvérisation cathodique, d'une évaporation sous vide ou d'un placage.

4. Un procédé d'analyse d'oxygène dans un gaz riche en hydrogène, qui comprend la détection de la différence de potentiel entre une anode et une cathode en contact avec un électrolyte exposé audit gaz, dans lequel il est prévu au moins une électrode supplémentaire en contact avec l'électrolyte et agencée pour intercepter des ions hydroxyle engendrés à la cathode, de façon à protéger l'anode contre le l'oxydation, caractérisé en ce qu'au moins une portion de surface de ladite électrode supplémentaire comprend de l'or, de l'argent ou du rhénium.
